# EUROPÄISCHE PATENTSCHRIFT

(11) **EP 3 247 459 B1**
(45) Veröffentlichungstag und Bekanntmachung des Hinweises auf die Patenterteilung: **29.08.2018**
(21) Anmeldenummer: 16703274.7
(22) Anmeldetag: 22.01.2016
(51) Int. Cl.: A61N 7/00, A46B 13/00, A61N 5/06

(54) **VORRICHTUNG ZUR ULTRASCHALLBEHANDLUNG OFFENER WUNDEN**
DEVICE FOR THE ULTRASOUND TREATMENT OF OPEN WOUNDS
DISPOSITIF POUR LE TRAITEMENT PAR ULTRASONS DES PLAIES OUVERTES

(30) Priorität: 22.01.2015 DE 102015201092
(43) Veröffentlichungstag der Anmeldung: 29.11.2017
(73) Patentinhaber: Frohwitter, Jens-Christian, 86356 Neusäß (DE)
(72) Erfinder: Frohwitter, Jens-Christian, 86356 Neusäß (DE)
(74) Vertreter: Himmelsbach, Mathias
(86) Internationale Anmeldenummer: PCT/EP2016/051379
(87) Internationale Veröffentlichungsnummer: WO 2016/116623

(56) Entgegenhaltungen:
- US-A1- 2004 030 254
- US-A1- 2005 278 877
- US-A1- 2007 219 470
- US-A1- 2009 177 125

## Beschreibung

Die Erfindung betrifft einen ultraschallunterstützenden Wundreiniger in Form eines Handgeräts. Die beschriebenen Verfahren dienen lediglich der Anschauung, und sind nicht Bestandteil der Erfindung.

Aus dem Stand der Technik sind unterschiedliche Arten und Vorrichtungen zur Wundreinigung bekannt. Aus US 2005/0278877 A1 ist bspw. eine Bürste zur Reinigung von chronischen Wunden bekannt. Die Bürste weist einen Drehantrieb zum rotatorischen Bewegen der Bürste auf. Ferner sind aus dem Stand der Technik ultraschallassistierte Wundreiniger bekannt. Bekannte Systeme, wie sie bspw. die Firmen Söring GmbH oder Misonix Inc. herstellen, weisen eine stationäre Einheit und ein über Schläuche verbundenes Handteil auf. In der stationären Einheit ist eine Pumpe zur Förderung einer Spül- und Koppelflüssigkeit sowie ein Ultraschallgenerator untergebracht. Das damit über Schläuche verbundene Handteil umfasst einen Reinigungsaufsatz aus Metall, welcher in Ultraschallschwingung versetzt wird, sowie eine Öffnung zum Austritt der von der stationären Basis geförderten Flüssigkeit in die Wunde.

Ferner sind auch Methoden des sogenannten Waterjet-Wunddebridements oder der chirurgischen Wundreinigung bekannt, welche sehr invasiv und somit nur in Ausnahmefällen, insbesondere bei großräumiger Gewebeentfernung, zur Anwendung kommen.

Die Methoden der mechanischen Wundreinigung, insbesondere durch Bürsten oder Tücher, stellen eine kostengünstige und häufig anwendbare Methode zur Wundreinigung dar. Sie haben jedoch den Nachteil, dass die Zerstörung bzw. Entfernung des sich in der Wunde befindlichen Biofilms nur sehr begrenzt ist, was zu einer häufigen Wiederholung der Anwendung führt. Die Ultraschallwundreinigung bietet hier insbesondere bei der Entfernung des Biofilms bessere Ergebnisse. Nachteilig an den bekannten Systemen sind jedoch die hohen Investitionskosten für die Geräte, die begrenzte Mobilität sowie die aufwendige Vorbereitung bzw. Nachbereitung des Behandlungsplatzes aufgrund der Keimverbreitung durch die während der Anwendung entstehenden Aerosole durch die zur Ultraschallübertragung und zur Spülung der Wunde zerstäubten Flüssigkeit.

Die Aufgabe der vorliegenden Erfindung besteht darin, eine Vorrichtung zur effektiven Wundreinigung bereitzustellen, welche kostengünstig sind, keiner aufwendigen Vor- und Nachbereitung bedarf und an jedem beliebigen Ort, an welchem sich der Patient befindet, anwendbar sind.

Die Aufgabe wird durch eine Vorrichtung gemäß Anspruch 1 gelöst. Weitere Fortbildungen der erfindungsgemäßen Lehre ergeben sich aus den jeweiligen Unteransprüchen.

Der erfindungsgemäße ultraschallunterstützte Wundreiniger in Form eines Handgerätes weist einen integrierten Vibrationsmotor, einen integrierten Ultraschallgenerator, eine integrierte Energiequelle für den Vibrationsmotor und den Ultraschallgenerator und einen vom Vibrationsmotor antreibbaren, auswechselbaren Reinigungsaufsatz zum abrasiven Entfernen von Wundbelägen auf. Der Reinigungsaufsatz weist einen Schallwandler, vorzugsweise ein Piezokristall, welcher mit dem Ultraschallgenerator in Verbindung steht, und eine Vorrichtung zum Verstärken und/oder Verteilen und/oder Weiterleiten der Ultraschallwellen auf. Der Schallwandler wandelt den vom Generator erzeugten, hochfrequenten Strom entsprechend seiner Amplitude und seiner Frequenz in Ultraschallschwingungen um.

Das Handgerät wird in diesem Zusammenhang insbesondere als tragbares, handgehaltenes und/oder mobiles Ultraschallreinigungsgerät angesehen. Ein solches Handgerät kommt insbesondere ohne eine Versorgungseinheit aus, welche nicht handgehalten ist und während der Behandlung mit dem handgehaltenen Geräten in Verbindung steht. Solche Versorgungseinheiten weisen insbesondere Aggregate wie Pumpen und/oder Schallgeneratoren auf. Somit ist das handgehaltene Instrument während der Benutzung, d. h. während der Wundreinigung, autark und einfach in der Handhabung. Durch die mobile Ausgestaltung als Handgerät besteht die Möglichkeit, dass die Patienten beispielsweise zu Hause von ihrem Hausarzt oder einem häuslichen Pflegedienst behandelt werden.

Das Handgerät umfasst eine Basis, in welcher der Vibrationsmotor, der Ultraschallgenerator und die Energiequelle integriert sind. Mit der Basis ist ein auswechselbarer Reinigungsaufsatz zum abrasiven Entfernen von Wundbelägen verbindbar. Der Reinigungsaufsatz weist vorzugsweise Borsten und/oder Lamellen zum mechanisch abrasiven Entfernen von Wundbelägen auf. Der vordere Bereich des Reinigungsaufsatzes, welcher durch die Borsten und/oder Lamellen gebildet wird, wird auch als Borstenkopf oder Reinigungskopf bezeichnet werden. Der Borstenkopf überragt dabei beim Wundreinigen die übrigen Bauteile des Reinigungsaufsatzes. Die Borsten und/oder Lamellen weisen eine derartige Länge auf, dass sie im Betrieb am weitesten überstehen bzw. am weitesten nach vorne abstehen und eine freiliegende Reinigungsfläche bilden. Dadurch wird gewährleistet, dass mit den Borsten/Lamellen unterschiedliche Wundtopographien, insbesondere ebene oder konkav ausgebildete Wundoberflächen, zuverlässig zu reinigen sind. Weiterhin soll vermieden werden, dass andere Bauteile des Reinigungsaufsatzes die Abrasion der Wundbeläge, insbesondere der Fibrinbeläge, durch den Reinigungskopf bzw. die Borsten und/oder Lamellen stören und vorzugsweise nur die Borsten und/oder Lamellen im Eingriff mit der Wundoberfläche sind.

Die Borsten und/oder Lamellen sind insbesondere zur Abtragung von Fibrinbelägen aus der Blutgerinnung geeignet und vorzugsweise aus Kunststoff oder Kunststofffasern ausgebildet. Weiter vorzugsweise weist der Reinigungsaufsatz einen oder mehrere Borstenkränze auf, welche beispielsweise konzentrisch zum Schallwandler und/oder der Vorrichtung zum Verstärken und/oder Verteilen und/oder Weiterleiten der Ultraschallwellen angeordnet sind. Bei der Ausbildung von mehr als einem Borstenkranz ist einer der Borstenkränze am Reinigungsaufsatz vorzugsweise die Vorrichtung zur Verstärkung und/oder Verteilung und/oder Weiterleitung der Ultraschallwellen derart umschließend angeordnet, dass ein Ausbreiten von Aerosolen unterbunden wird. Somit wird durch den Borstenkranz ein "Schutzschild" oder eine Kontaminationssperre, insbesondere gegen die Ausbreitung von mit Bakterien kontaminierten Aerosolen, gebildet, welche eine Kontamination angrenzender Hautbereiche sowie des Umfelds verhindert. Zur Reinigung von tiefen, kanalförmigen Wunden ist der Borstenkopf vorzugsweise in der Art eines Pfeifenreinigers ausgebildet, bei welchem die Borsten radial abstehen.

Der Reinigungskopf umfasst weiter vorzugsweise eine Sonotrode. Die Sonotrode verstärkt dabei die Amplitude der Ultraschallwellen und dient als Überträger der Schallwellen. Die Sonotrodenform sowie die Oberfläche der Sonotrode variieren je nach Anwendungsbereich. Beispielsweise kann durch die Sonotrodenform eine beispielsweise radiale oder axiale Abstrahlung der Ultraschallwellen in Bezug auf die Längsausstreckung des Reinigungsaufsatzes erfolgen. Je nach Ausgestaltung des Schallverteilers und/oder der Sonotrode sind die Ultraschallwellen fokusierbar oder defokusierbar. Der Reinigungsaufsatz ist durch Gestaltung des Schallverteilers und/oder der Sonotrode an unterschiedliche Anwendungen anpassbar.

Der Vibrationsmotor arbeitet vorzugsweise mit einer Frequenz von 100 bis 5000 Hz, weiter vorzugsweise mit einer Frequenz von 100 bis 500 Hz, welche weitere vorzugsweise einstellbar ist. Der Vibrationsmotor ist in der Basis des Handgerätes angeordnet und ist derart ausgebildet, dass der auswechselbare Reinigungsaufsatz und insbesondere der Reinigungskopf samt Borsten/Lamellen vom Vibrationsmotor antreibbar ist. Die Verbindung von Basis und Reinigungsaufsatz erfolgt vorzugsweise über eine formschlüssige Steckverbindung und verfügt über eine Antriebsmechanik, wie sie beispielsweise bei elektrischen Zahnbürsten verwendet wird. Der Reinigungsaufsatz ist dabei insbesondere derart ausgebildet, dass die Borsten und/oder Lamellen vom Vibrationsmotor antreibbar, insbesondere intermittierend rotatorisch antreibbar sind.

Der Ultraschallgenerator erzeugt vorzugsweise eine Frequenz zwischen 20 kHz und 2 MHz, weiter vorzugsweise 20 kHz bis 1 MHz, weiter vorzugsweise 20 kHz bis 500 kHz, weiter vorzugsweise 20 kHz bis 100 kHz und weiter vorzugsweise 20 kHz bis 50 kHz. Die vom Ultraschallgenerator erzeugte Frequenz und/oder die Amplitude ist insbesondere einstellbar. Die in die Wunde eingebrachten Ultraschallwellen bewirken Mikrokavitation in einem Übertragungsmedium wie bspw. einer Spülflüssigkeit oder einem Gel, so dass mechanische Kräfte den in der Wunde vorhanden Biofilm aufsprengen. Dadurch werden die im Biofilm enthaltenen Bakterienbeläge abgetragen. Ferner wird durch die Aufsprengung des Biofilms die Wirksamkeit von Desinfektionslösungen verbessert. Das Ultraschallsignal bewirkt darüber hinaus eine Stimulation des Metabolismus der Bakterien, so dass die Desinfektionsmittel besser von den Bakterien absorbiert werden und besser wirken. Auch wird das Anhaftvermögen der Bakterien untereinander eingeschränkt. Dies wirkt insbesondere der Neubildung des Biofilms entgegen.

Der ultraschallunterstützte Wundreiniger weist vorzugsweise eine Reinigungsaufsatzerkennungsvorrichtung auf. Die Reinigungsköpfe sind dabei vorzugsweise über eine softwarebasierte Codierung mit dem Handapparat verknüpft. Dadurch sind insbesondere die Art des verwendeten Reinigungsaufsatzes sowie dessen Funktionsumfang und die technischen Randbedingungen wie beispielsweise die Borstenform von der Basis erkennbar. Somit kann der Reinigungsaufsatz optimal betrieben werden. Ferner ist durch die Erkennungsvorrichtung auch die Mehrfachverwendung von Reinigungsaufsätzen unterbindbar. Insbesondere durch die Kennzeichnung des Reinigungsaufsatzes, beispielsweise in einem Speicher im Reinigungsaufsatz, wird ein erneutes Verwenden des Reinigungsaufsatzes unterbunden. Eine Kennzeichnung als benutzt erfolgt in der Regel bei erstmaliger Inbetriebnahme des Wundreinigers mit dem jeweiligen Reinigungsaufsatz. Auch die Verwendung von nicht geeigneten Reinigungsaufsätzen ist durch eine derartige Erkennungsvorrichtung unterbindbar. Vorzugsweise erfolgt die Kommunikation zwischen Basis und Reinigungsaufsatz verschlüsselt. Die Kommunikation zwischen Basis und Reinigungsaufsatz kann kabelgebunden oder alternativ über Funk, bspw. gemäß RFID-Technik, erfolgen.

Weiter vorzugsweise umfasst der Reinigungsaufsatz eine UV-Diode zur Bestrahlung der Wunde während der Behandlung mit UV-Licht. Durch die Bestrahlung UV-Licht tritt eine zusätzliche, keimabtötende Wirkung ein, welche die Wundreinigung und auch die Wundheilung unterstützt.

Der Wundreiniger umfasst vorzugsweise eine induktive Ladevorrichtung zum Laden des als Energiequelle verwendeten Akkumulators. Dadurch ist die Energiequelle des Wundreinigers in einfacher Art und Weise aufladbar. Hierzu wird in der Regel eine Basisstation verwendet, welche über ein Netzteil verfügt. Während der Verwendung des Wundreinigers wird diese externe Basisstation nicht benötigt bzw. der Wundreiniger wird von der Basisstation gelöst, so dass es sich um einen kompakten und handgehaltenen Wundreiniger handelt. Der Wundreiniger in Form eines Handgeräts hat lediglich ein Gewicht in der Größenordnung von etwa 100 g und kann somit in einfacher Art und Weise zum behandelnden Patienten mitgenommen werden.

Der Wundreiniger weist insbesondere eine Steuerungselektronik zur Ansteuerung der verschiedenen Funktionen wie Ultraschallgeneration, Vibrationserzeugung und ggf. UV-Lichtbehandlung auf. Die Steuerungselektronik umfasst dabei vorzugsweise einen Druck-Taster bzw. Schalter zum Ein- und Ausschalten sowie zum Vorwählen verschiedener Betriebsmodi wie beispielsweise unterschiedlicher Frequenzen oder der Auswahl einzelner Funktionen. Die aktivierten Funktionen und/oder ausgewählten Frequenzen werden dabei vorzugsweise über LEDs oder ein Display angezeigt. Die Steuerung regelt dabei die in der Basis angeordneten Module wie beispielsweise Ultraschallgenerator oder Vibrationsmotor an. Die elektrischen Schwingungen des Ultraschallgenerators werden über elektrische Leitungen zum Schallwandler im Reinigungsaufsatz übertragen. Des Weiteren umfassen die Reinigungsaufsatzerkennung wie auch die UV-Diode gegebenenfalls elektrische Leitungen von der Basis in den Reinigungsaufsatz. Diese werden über elektrische Kontakte an der Basis bzw. am Reinigungsaufsatz übertragen.

Der Reinigungsaufsatz ist ferner mit einem Einmal-Schutzüberzug ausbildbar, welcher über die Basis des Wundreinigers gestreift wird. Dadurch wird die Basis vor Kontamination während der Behandlung geschützt, und der Überzug wird nach Beendigung der Behandlung zusammen mit dem Reinigungsaufsatz entsorgt.

Bei einem Verfahren zum Reinigen von Wunden wird ein Reinigungsaufsatz mit Borsten zum abrasiven Entfernen von Wundbelägen über einen Vibrationsmotor bewegt. Der Reinigungsaufsatz wird über einen Ultraschallgenerator in Schwingung versetzt, und die Schwingungen werden zur Unterstützung der Ablösung von Wundbelägen über ein Übertragungsmedium in die Wunde übertragen.

Als Übertragungsmedium, insbesondere für die Schallwellen vom Reinigungsaufsatz in die Wunde, wird vorzugsweise ein wasserbasiertes Medium oder ein Gel verwendet. Das Übertragungsmedium wird vor Beginn der Wundreinigung entweder auf die zu reinigende Wunde und/oder auf den Borstenkopf aufgetragen. Auch kann der Reinigungsaufsatz ein Gelreservoir aufweisen, welches sich während der Behandlung kontinuierlich entleert. Durch die Bewegung der Borsten durch den Vibrationsmotor werden Wundbeläge abrasiv im Groben entfernt. Während des Verfahrens der Wundreinigung sollte ein regelmäßiges Reinigen des Borstenkopfes erfolgen. Auch besteht die Möglichkeit, verunreinigtes Übertragungsmedium auszutauschen bzw. neues Übertragungsmedium hinzuzugeben. Durch das Übertragungsmedium werden die Ultraschallwellen zur Wundoberfläche übertragen, und es entsteht im Übertragungsmedium Kavitation. Durch das Zerplatzen der Kavitationsbläschen wird die Wundoberfläche, insbesondere der Biofilm auf der Wundoberfläche, aufgesprengt und entfernt.

Bei der Behandlung von chronischen, insbesondere offenen Wunden kann es zu Schmerzen für den Patienten kommen. Aus diesem Grund kann das Übertragungsmedium schmerzlindernde oder betäubende Wirkstoffe enthalten. Ferner können dem Gel auch wundbelagslösende und/oder desinfizierende Substanzen beigemischt sein. Als Lösungsmittel und Desinfektionsmittel werden insbesondere Polyhexamethylenbiguanid, welches auch als Polyhexanid oder PHMB bekannt ist, Silberlösungen und/oder Silbergels verwendet. Zusätzlich oder alternativ kann bei dem Verfahren die desinfizierende Wirkung durch UV-Licht erhöht werden.

Der Reinigungsaufsatz und insbesondere die Borsten können vom Vibrationsmotor rotatorisch oder linear bewegt werden. Die Bewegung erfolgt dabei vorzugsweise intermittierend rotatorisch oder linear. Der Vibrationsmotor generiert vorzugsweise eine Vibration der Borsten mit einer Frequenz von 100 bis 5000 Hz. Der Ultraschallgenerator generiert bei dem Verfahren eine Frequenz zwischen 20 kHz bis 2 MHz, vorzugsweise 20 kHz bis 1 MHz, weiter vorzugsweise 20 kHz bis 500 kHz weiter vorzugsweise 20 kHz bis 100 kHz und weiter vorzugsweise 20 kHz bis 50 kHz. Die Frequenzen des Vibrationsmotors und/oder des Ultraschallgenerators sind vorzugsweise einstellbar.

Das Verfahren wird vorzugsweise mit einem, wie oben beschriebenen, erfindungsgemäßen ultraschallunterstützten Wundreiniger in Art eines Handgerätes durchgeführt.

Der erfindungsgemäße portable ultraschallunterstützte Wundreiniger und das Verfahren zum Reinigen von Wunden stellen eine effektive Wundreinigung durch Kombination der abrasiven Abtragung von Fibrinbelägen über die Borsten/Lamellen und durch das Aufsprengen des Biofilms durch die vom Ultraschall erzeugte Mikrokavitation bereit. Gemäß der Erfindung werden die Schwingungen sowohl des Vibrationsmotors als auch des Ultraschallgenerators / Schallwandlers, vorzugsweise zeitparallel, zur Reinigung genutzt. Es handelt sich somit um ein selektives und gewebeschonendes Verfahren zur Behandlung von Wunden, welches nahezu unbegrenzt oft durchgeführt werden kann. Die Wundreinigung und auch die Wundheilung können dabei insbesondere durch keimabtötende UV-Strahlen oder Substanzen im Übertragungsmedium bzw. Gel unterstützt werden. Durch die Ausbildung als autonomer, handgehaltener Applikator ist der Wundreiniger für eine große Patientenzahl verfügbar bzw. kann bei diesen angewendet werden.

Weitere Merkmale und Vorteile der Erfindung ergeben sich aus den nachfolgend beschriebenen Ausführungsbeispielen in Verbindung mit den Figuren. Dabei zeigt
- Figur 1:: einen ultraschallunterstützten Wundreiniger in Form eines Handgerätes gemäß der Erfindung,
- Figur 2:: einen alternativen Reinigungsaufsatz eines erfindungsgemäßen Wundreinigers, und
- Figur 3:: einen Teil eines weiteren, alternativen Reinigungsaufsatzes gemäß der Erfindung.

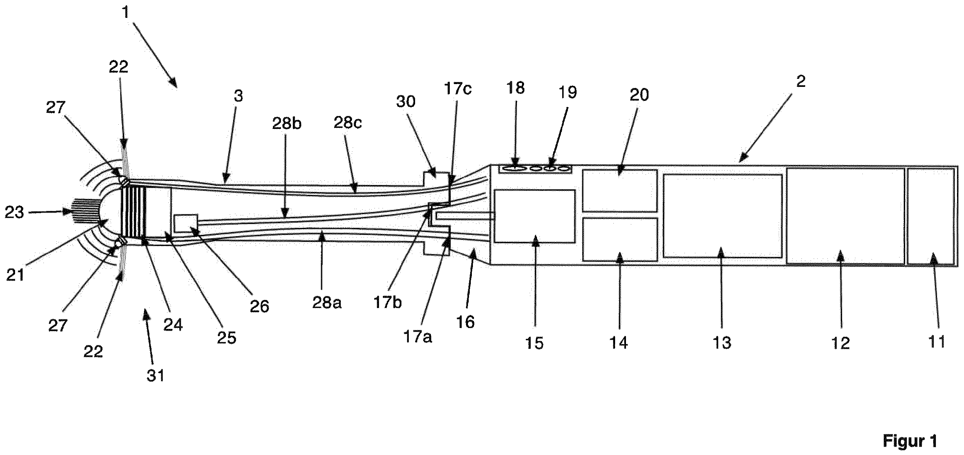

Figur 1 zeigt einen erfindungsgemäßen, ultraschallunterstützten Wundreiniger 1 in Form eines Handgerätes. Der Wundreiniger umfasst eine Basis 2 und einen auswechselbaren Reinigungsaufsatz 3. Die Basis 2 umfasst einen Akku 12 sowie eine induktive Ladevorrichtung 11 zum Laden des Akkus 12. Ferner umfasst die Basis 2 einen Ultraschallgenerator 13, d. h. einen Generator zur Erzeugung eines hochfrequenten Stroms. Die Frequenz des hochfrequenten Stroms ist über den Bedientaster 18 zwischen 20 kHz und 2 MHz einstellbar. Die eingestellte Frequenz wird über das optische Anzeigeelement 19 wiedergegeben. Der vom Ultraschallgenerator 13 erzeugte hochfrequente Strom wird über Kontaktpunkte 17a, 29a in der Basis 2 und im Reinigungsaufsatz 3 und eine elektrische Leitung 28a an einen Schallwandler 24 geleitet. Der Schallwandler 24 ist im vorderen Bereich des Reinigungsaufsatzes 3, dem Borstenkopf 31, angeordnet. Der Schallwandler 24 wandelt den hochfrequenten Strom in hochfrequente Ultraschallwellen um. Als Schallwandler 24 wird ein Piezokristall verwendet. Der Schallwandler 24 ist mit einer Gegenmasse 25 verbunden, welche als Trägheitsmasse zur Erzeugung der Ultraschallwellen verwendet wird und somit zur gerichteten Ausbreitung der Schallwellen dient. Darüber hinaus werden die Schallwellen über eine Sonotrode 21 in die zu behandelnde Wunde übertragen.

Die Basis 2 umfasst ferner einen Vibrationsmotor 15. Die Ausgangswelle des Vibrationsmotors 15 ist über einen Steckverbinder 30 formschlüssig mit dem Reinigungsaufsatz 3 verbunden, um Borsten 22, 23 des Reinigungsaufsatzes über eine nicht gezeigte Mechanik in eine intermittierende Rotations- oder Linearbewegung zu versetzen.

Der Wundreiniger 1 verfügt darüber hinaus über ein System zur Erkennung des aufgesetzten Reinigungsaufsatzes 3. Hierzu steht ein Schreib-Lese-Speicher 14 über Kontaktpunkte 17b, 29b und einen elektrischen Leiter 28b mit einem Chip 26 im Reinigungsaufsatz 3 in Verbindung. Durch die Reinigungsaufsatz-Erkennung wird gewährleistet, dass von der Basis 2 erkannt wird, um was für eine Art von Reinigungsaufsatz 3 es sich handelt und welche technischen Daten, wie beispielsweise Art der Borsten, Funktionen des Reinigungsaufsatzes sowie dessen technische Parameter dieser aufweist. Dadurch wird zum einen ermöglicht, dass der Reinigungsaufsatz 3 entsprechend seinen Randbedingungen angesteuert wird. Zum anderen wird durch die Reinigungsaufsatz-Erkennung gewährleistet, dass jeder Bürstenaufsatz nur einmal verwendet wird, um eine Übertragung von Keimen zu unterbinden. Ferner kann durch die Erkennung des Reinigungsaufsatzes die Verwendung von nicht freigegebenen oder falschen Reinigungsaufsätzen unterbunden werden. Um die Verwendung eines bereits benützten Reinigungsaufsatzes auch nicht in Verbindung mit einer zweiten Basis 2 zu ermöglichen, wird die Verwendung auf dem Chip 26 des Reinigungsaufsatzes 3 gespeichert.

Der Reinigungsaufsatz 3 weist in seinem vorderen Bereich zudem UV-Dioden 27 auf. Die UV-Dioden strahlen während der Behandlung, d. h. im Betrieb des Wundreinigers 1, UV-Strahlung zur Keimabtötung aus. Die UV-Dioden 27 sind über den elektrischen Leiter 28c sowie die Kontaktpunkte 29c bzw. 17c mit der Basis 2 verbunden und werden von dieser entsprechend gesteuert.

Zur Steuerung der Funktionen des Wundreinigers 1 wie Ultraschallerzeugung, Vibration, UV-Abstrahlung und Reinigungsaufsatz-Erkennung verfügt die Basis 2 über eine Steuerplatine 20. Diese ist mit den einzelnen Modulen wie Ultraschallgenerator 13, Vibrationsmotor 15 verbunden. Darüber hinaus ist die Steuerplatine 20 mit dem Bedientaster 18 sowie einem Display als optisches Anzeigeelement 19 verbunden, um die verschiedenen Funktionen und Modi einzustellen bzw. den momentanen Betriebsmodus anzuzeigen.

Der Reinigungsaufsatz 3 weist in seinem vorderen Bereich einen Borstenkranz 22 auf, welcher den Schallwandler bzw. die Sonotrode 21 umringt und die Ausbreitung von Aerosolen unterbindet. Der Borstenkranz 22 bildet sozusagen ein Schutzschild, welches die Kontamination der Umgebung verhindert. Die zentral am Reinigungskopf angeordneten Borsten 23 sind zur mechanischen bzw. abrasiven Entfernung von Wundbelägen. Bei Verwendung des Wundreinigers 1, insbesondere zur Reinigung von chronischen Wunden, wird zunächst als steriles Einmalprodukt verpackter Reinigungsaufsatz 3 auf die Basis 2 aufgesteckt. Durch die Reinigungsaufsatz-Erkennungselektronik wird der Reinigungsaufsatz erkannt, und es wird beurteilt, ob es sich um einen geeigneten Reinigungsaufsatz 3 handelt. Über den Bedientaster 18 werden die Betriebsparameter wie beispielsweise Frequenz und Amplitude des Ultraschallgenerators, Frequenz des Vibrationsmotors und die Verwendung der UV-Diode vorgewählt.

Die Wunde wird mit einem Gel vorbehandelt, d. h. es wird ein schmerzlinderndes bzw. betäubendes Gel mit desinfizierenden Substanzen in die Wunde eingebracht. Nach einer vordefinierten Einwirkzeit wird die Wunde mit dem Wundreiniger 1 gereinigt. Zum einen werden dabei über mechanische Abrasion mittels der Borsten 23 Fibrinbeläge, d. h. Produkte aus der Blutgerinnung, aus der Wunde abgetragen. Zum anderen wird durch den Ultraschall Mikrokavitation in der Wunde erzeugt, wodurch der Biofilm mit seinen Bakterien aufgesprengt wird. Nach Inbetriebnahme des Reinigungsaufsatzes 3 wird dieser im Chip 26 als benutzt markiert, so dass eine weitere Verwendung desselben Reinigungsaufsatzes 3 unterbunden ist. Dies dient auch der Prävention irrtümlicher Wiederverwendung am nächsten Patienten und somit der Kontamination. Der Reinigungsaufsatz 3 wird somit nach der einmaligen Verwendung zur Reinigung einer Wunde entsorgt.

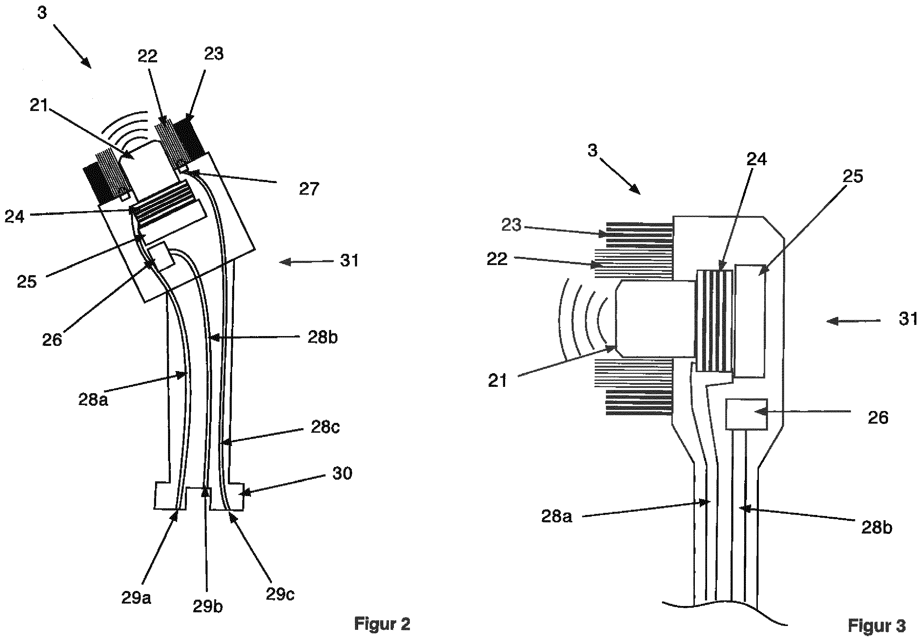

Figuren 2 und 3 zeigen alternative Ausgestaltungen des Reinigungsaufsatzes 3. Die Ausbildung der Reinigungsaufsätze 3 unterscheidet sich je nach Art der zur reinigenden Wunde. Für tieferliegende Wundgebiete oder Wundtaschen weist der Reinigungsaufsatz 3 eine entsprechende Länge auf und ist gegebenenfalls flexibel, d. h. biegbar an die Wundform anpassbar ausgestaltet. Der in Figur 2 gezeigte Reinigungsaufsatz weist abweichend von dem Reinigungsaufsatz 3 gemäß Figur 1 zwei Borstenkränze 22, 23 auf. Beide Borstenkränze 22, 23 sind konzentrisch um die Sonotrode herum angeordnet und überragen diese in Längsrichtung der Borsten, sodass eine freiliegende Reinigungsfläche gebildet wird. Die Borsten 22, 23 sowie der Borstenkopf 31 ist zur Hauptausrichtung des Reinigungsaufsatzes 3 um einen vordefinierten Winkel geneigt.

Die Ausführungsform gemäß Figur 3 unterscheidet sich darin von der Ausführungsform gemäß Figur 2, dass der Reinigungsaufsatz keine UV-Dioden zur Keimabtötung aufweist. Darüber hinaus ist der Borstenkopf 31 um 90° zur Hauptausdehnungsrichtung des Reinigungsaufsatzes 3 geneigt.

### Bezugszeichenliste:

- 1: Wundreiniger
- 2: Basis
- 3: Reinigungsaufsatz
- 11: Induktive Ladevorrichtung
- 12: Akku
- 13: Ultraschallgenerator
- 14: Schreib-Lese-Speicher
- 15: Vibrationsmotor
- 16: Steckvorrichtung
- 17a, b, c: Kontaktpunkte
- 18: Schalter
- 19: Optische Anzeige
- 20: Steuerplatine
- 21: Sonotrode
- 22: Erster Borstenkranz
- 23: Zweiter Borstenkranz
- 24: Schallwandler
- 25: Gegenmasse
- 26: Chip
- 27: UV-Diode
- 28a, b, c: Elektrischer Leiter
- 29a, b, c: Kontaktpunkte
- 30: Steckverbinder
- 31: Borstenkopf

## Patentansprüche

1. Ultraschallunterstützter Wundreiniger (1) in Form eines Handgerätes mit integriertem Vibrationsmotor (15), integrierter Energiequelle (12) für den Vibrationsmotor (15) und einem vom Vibrationsmotor (15) antreibbaren, auswechselbaren Reinigungsaufsatz (3) zum abrasiven Entfernen von Wundbelägen, wobei der Reinigungsaufsatz (3) Borsten und/oder Lamellen (22, 23) zum abrasiven Entfernen aufweist
**dadurch gekennzeichnet, dass**
der ultraschallunterstützte Wundreiniger (1) ferner einen integrierten Ultraschallgenerator (13) und die integrierte Energiequelle (12) für den Ultraschallgenerator (13) aufweist, wobei der Reinigungsaufsatz (3) einen Schallwandler (24), welcher derart ausgebildet ist, dass er die vom Ultraschallgenerator (13) erzeugten elektrischen Schwingungen in Schallwellen wandelt, sowie eine Vorrichtung (21) zur Verstärkung und/oder Verteilung und/oder Weiterleitung der Ultraschallwellen aufweist.

2. Ultraschallunterstützter Wundreiniger (1) nach Anspruch 1, **dadurch gekennzeichnet, dass** die Borsten und/oder Lamellen (22, 23) derart ausgebildet sind, dass sie beim Wundreinigen über die übrigen Bauteile des Reinigungsaufsatzes (3) hinausragen.

3. Ultraschallunterstützter Wundreiniger (1) nach Anspruch 1 oder 2, **dadurch gekennzeichnet, dass** der Reinigungsaufsatz (3) einen Borstenkranz (23) zum abrasiven Entfernen aufweist.

4. Ultraschallunterstützter Wundreiniger (1) nach Anspruch 3, **dadurch gekennzeichnet, dass** der Reinigungsaufsatz (3) einen weiteren Borstenkranz (22) aufweist, welcher die Vorrichtung (21) zur Verstärkung und/oder Verteilung und/oder Weiterleiten der Ultraschallwellen derart umschließend angeordnet ist, dass ein Ausbreiten von Aerosolen unterbunden wird.

5. Ultraschallunterstützter Wundreiniger (1) nach einem der vorherigen Ansprüche, **dadurch gekennzeichnet, dass** das Handgerät eine Reinigungsaufsatz-Erkennungsvorrichtung aufweist.

6. Ultraschallunterstützter Wundreiniger (1) nach einem der vorherigen Ansprüche, **dadurch gekennzeichnet, dass** der Vibrationsmotor (15) mit einer einstellbaren Frequenz von 100 bis 5000 Hz arbeitet.

7. Ultraschallunterstützter Wundreiniger (1) nach einem der vorherigen Ansprüche, **dadurch gekennzeichnet, dass** der Ultraschallgenerator (13) eine Frequenz zwischen 20 kHz bis 2 MHz, vorzugsweise 20 kHz bis 1 MHz, weiter vorzugsweise 20 kHz bis 500 kHz, weiter vorzugsweise 20 kHz bis 100 kHz, weiter vorzugsweise 20 kHz bis 50 kHz erzeugt.

8. Ultraschallunterstützter Wundreiniger (1) nach einem der vorherigen Ansprüche, **dadurch gekennzeichnet, dass** die vom Ultraschallgenerator (13) erzeugte Frequenz einstellbar ist.

9. Ultraschallunterstützter Wundreiniger (1) nach einem der vorherigen Ansprüche, **dadurch gekennzeichnet, dass** die Energiequelle (12) ein Akkumulator ist, welcher vorzugsweise über eine induktive Ladevorrichtung (11) aufladbar ist.

## Claims

1. Ultrasound-assisted wound cleaner (1) in the form of a handheld appliance with an integrated vibration motor (15), an integrated energy source (12) for the vibration motor (15), and a replaceable cleaning attachment (3) which can be driven by the vibration motor (15) for the purpose of abrasively removing wound coatings, wherein the cleaning attachment (3) has bristles and/or lamellas (22, 23) for the abrasive removal,
**characterized in that**
the ultrasound-assisted wound cleaner (1) also has an integrated ultrasound generator (13) and the integrated energy source (12) for the ultrasound generator (13), wherein the cleaning attachment (3) has an acoustic transducer (24), which is designed to convert the electric oscillations generated by the ultrasound generator (13) into sound waves, and a device (21) for strengthening and/or distributing and/or transmitting the ultrasound waves.

2. Ultrasound-assisted wound cleaner (1) according to Claim 1, **characterized in that** the bristles and/or lamellas (22, 23) are designed in such a way that they protrude past the other structural parts of the cleaning attachment (3) during the wound cleaning.

3. Ultrasound-assisted wound cleaner (1) according to Claim 1 or 2,
**characterized in that** the cleaning attachment (3) has a bristle ring (23) for the abrasive removal.

4. Ultrasound-assisted wound cleaner (1) according to Claim 3, **characterized in that** the cleaning attachment (3) has a further bristle ring (22) which is arranged surrounding the device (21) for strengthening and/or distributing and/or transmitting the ultrasound waves, in such a way that a propagation of aerosols is suppressed.

5. Ultrasound-assisted wound cleaner (1) according to one of the preceding claims, **characterized in that** the handheld appliance has a cleaning attachment identification device.

6. Ultrasound-assisted wound cleaner (1) according to one of the preceding claims, **characterized in that** the vibration motor (15) operates with an adjustable frequency of 100 to 5000 Hz.

7. Ultrasound-assisted wound cleaner (1) according to one of the preceding claims, **characterized in that** the ultrasound generator (13) generates a frequency of 20 kHz to 2 MHz, preferably 20 kHz to 1 MHz, more preferably 20 kHz to 500 kHz, more preferably 20 kHz to 100 kHz, more preferably 20 kHz to 50 kHz.

8. Ultrasound-assisted wound cleaner (1) according to one of the preceding claims, **characterized in that** the frequency generated by the ultrasound generator (13) is adjustable.

9. Ultrasound-assisted wound cleaner (1) according to one of the preceding claims, **characterized in that** the energy source (12) is an accumulator which can preferably be charged via an inductive charger (11).

## Revendications

1. Dispositif de nettoyage de plaie par ultrasons (1) sous la forme d'un appareil manuel avec un moteur vibrant intégré (15), une source d'énergie intégrée (12) pour le moteur vibrant (15) et un embout de nettoyage remplaçable (3), pouvant être entraîné par le moteur vibrant (15) pour enlever des croûtes par abrasion, dans lequel l'embout de nettoyage (3) présente des poils ou des lamelles (22, 23) pour l'enlèvement par abrasion, **caractérisé en ce que** le dispositif de nettoyage de plaie par ultrasons (1) présente en outre un générateur d'ultrasons intégré (13) et la source d'énergie intégrée (12) pour le générateur d'ultrasons (13), dans lequel l'embout de nettoyage (3) présente un transducteur acoustique (24), qui est configuré de façon à convertir les oscillations électriques produites par le générateur d'ultrasons (13) en ondes sonores, ainsi qu'un dispositif (21) pour amplifier et/ou répartir et/ou retransmettre les ondes ultrasonores.

2. Dispositif de nettoyage de plaie par ultrasons (1) selon la revendication 1, **caractérisé en ce que** les poils et/ou les lamelles (22, 23) sont réalisés de telle manière qu'ils/elles dépassent les autres composants de l'embout de nettoyage (3) lors du nettoyage d'une plaie.

3. Dispositif de nettoyage de plaie par ultrasons (1) selon la revendication 1 ou 2, caractérisé en que l'embout de nettoyage (3) présente une couronne de poils (23) pour l'enlèvement par abrasion.

4. Dispositif de nettoyage de plaie par ultrasons (1) selon la revendication 3, **caractérisé en ce que** l'embout de nettoyage (3) présente une autre couronne de poils (22), qui est disposée autour du dispositif (21) pour amplifier et/ou répartir et/ou retransmettre les ondes ultrasonores, de telle manière qu'une diffusion d'aérosols soit empêchée.

5. Dispositif de nettoyage de plaie par ultrasons (1) selon l'une quelconque des revendications précédentes, **caractérisé en ce que** l'appareil manuel présente un dispositif de reconnaissance d'embout de nettoyage.

6. Dispositif de nettoyage de plaie par ultrasons (1) selon l'une quelconque des revendications précédentes, **caractérisé en ce que** le moteur vibrant (15) travaille avec une fréquence réglable de 100 à 5000 Hz.

7. Dispositif de nettoyage de plaie par ultrasons (1) selon l'une quelconque des revendications précédentes, **caractérisé en ce que** le générateur d'ultrasons (13) produit une fréquence comprise entre 20 kHz et 2 MHz, de préférence entre 20 kHz et 1 MHz, de préférence encore entre 20 kHz et 500 kHz, de préférence encore entre 20 kHz et 100 kHz, et de préférence encore entre 20 kHz et 50 kHz.

8. Dispositif de nettoyage de plaie par ultrasons (1) selon l'une quelconque des revendications précédentes, **caractérisé en ce que** la fréquence produite par le générateur d'ultrasons (13) est réglable.

9. Dispositif de nettoyage de plaie par ultrasons (1) selon l'une quelconque des revendications précédentes, **caractérisé en ce que** la source d'énergie (12) est un accumulateur, qui peut être chargé de préférence au moyen d'un dispositif de chargement par induction (11).
